# EUROPEAN PATENT APPLICATION

(11) **EP 1 952 758 A1**
(43) Date of publication of application: **06.08.2008**
(21) Application number: 06823301.4
(22) Date of filing: 10.11.2006
(51) Int. Cl.: A61B 5/022

(54) **ELECTRONIC SPHYGMOMANOMETER AND METHOD FOR REPORTING THE NUMBER OF MEASUREMENTS IN THE ELECTRONIC SPHYGMOMANOMETER**

(30) Priority: 17.11.2005 JP 2005332604
(71) Applicant: Omron Healthcare Co., Ltd., Kyoto 615-0084 (JP)
(72) Inventor: KISHIMOTO, Hiroshi, Kyoto-shi, Kyoto 615-0084 (JP); SAWANOI, Yukiya, Kyoto-shi, Kyoto 615-0084 (JP); MATSUMURA, Naomi, Kyoto-shi, Kyoto 615-0084 (JP); TANAKA, Takahide, Kyoto-shi, Kyoto 615-0084 (JP)
(74) Representative: Wilhelms · Kilian & Partner Patentanwälte
(86) International application number: PCT/JP2006/322479
(87) International publication number: WO 2007/058128

(57) **Abstract**

For each blood pressure measurement, measured blood pressure data output by a blood pressure calculation unit (201) is stored in a memory (13). On this occasion, the number of times of measurement is incremented by a number of times of measurement update unit (261), and data on the incremented number of times of measurement is stored in the memory (13). Based on information read from the memory (13), a number of times display data generation unit (271) generates display data for a count result of the number of times of blood pressure measurement, and supplies the generated display data to a display control unit (241). Based on the supplied data, the display control unit (241) displays the count result of the number of times of blood pressure measurement on a display unit (2).

## Description

### TECHNICAL FIELD

The present invention relates to an electronic blood pressure monitor and a method of informing the number of times of measurement in the electronic blood pressure monitor, and particularly to an electronic blood pressure monitor having a function of displaying stored measurement data and a method of informing the number of times of measurement in the electronic blood pressure monitor.

### BACKGROUND ART

In recent years, lifestyle-related diseases due to high blood pressure have become widespread, and a blood pressure value has been used as an indicator for health management. Blood pressure management is important, and it is useful for better blood pressure management to accurately measure blood pressure every day and manage changes in blood pressure as a trend.

It is recommended to measure blood pressure at least once or several times in the morning (after waking up) and in the night (before sleeping) of a day, calculate an average value, and manage blood pressure. Examples of conventional techniques include those described below.

In a blood pressure monitor disclosed in Japanese Patent Laying-Open No. 2002-102184, blood pressure measurement performed by pressurizing and depressurizing a cuff (a pressurizing belt) is automatically repeated for two or more cycles, preferably for three cycles. Each average value of the highest blood pressure (systolic blood pressure), the lowest blood pressure (diastolic blood pressure), and a pulse rate per minute measured for each cycle, and the greatest deviation between the average value and the measured value thereof are automatically calculated and displayed when blood pressure measurement for the last cycle is finished. The number of cycles to repeat the measurement can be set beforehand.

In a blood pressure monitor disclosed in Japanese Patent Laying-Open No. 2001-070260, a measurement time point is set, and either an operation to inform a subject to measure blood pressure or an operation to automatically perform blood pressure measurement can be selectively carried out at the set measurement time point. The measurement time point that can be set includes a time point to start measurement and a measurement time interval. The measurement time point is externally set by communication means. The blood pressure monitor also has means receiving that the subject was awake during automatic measurement.
Patent Document 1: Japanese Patent Laying-Open No. 2002-102184
Patent Document 2: Japanese Patent Laying-Open No. 2001-070260

### DISCLOSURE OF THE INVENTION

### PROBLEMS TO BE SOLVED BY THE INVENTION

With a conventional electronic blood pressure monitor, it is not easy to confirm the number of times of blood pressure measurement performed on the day, during the week, or the like, or the remaining number of times of blood pressure measurement to be performed on the day, during the week, or the like. Further, incorporation of a high value-added feature such as automatic measurement leads to an increase in the cost of the monitor. Incorporation of an advanced feature also results in complicated operations and settings, which makes elderly people, for example, feel difficult to use the monitor itself, thus leading to poor compliance with blood pressure measurement.

Accordingly, one object of the present invention is to provide an electronic blood pressure monitor informing a count result of the number of times of blood pressure measurement, and a method of informing the number of times of measurement in the electronic blood pressure monitor.

### MEANS FOR SOLVING THE PROBLEMS

An electronic blood pressure monitor in accordance with an aspect of the present invention includes: a blood pressure measurement unit having a cuff to be attached to a blood pressure measurement region, a pressurization and depressurization unit adjusting pressure to be applied to the cuff, a pressure detection unit detecting pressure inside the cuff adjusted by the pressurization and depressurization unit, and a blood pressure calculation unit calculating blood pressure based on the pressure detected by the pressure detection unit; a number of times of measurement count unit counting a number of times of blood pressure measurement performed by the blood pressure measurement unit; and an informing unit informing a count result counted by the number of times of measurement count unit.

Accordingly, since the count result of the number of times of blood pressure measurement is informed, a subject can utilize the result to confirm the number of times of measurement or to obtain guidance on the required number of times of measurement.

Preferably, the number of times of measurement count unit counts the number of times of blood pressure measurement for each day or for each week. Accordingly, a count result of the number of times of measurement performed on the day, during the week, or the like can be confirmed, which is useful for continuous measurement control on a daily basis necessary for blood pressure management.

Preferably, the electronic blood pressure monitor further includes a timer for measuring time, and the number of times of measurement count unit counts the number of times of blood pressure measurement performed by the blood pressure measurement unit in a prescribed time slot measured by the timer.

Preferably, the number of times of measurement count unit counts the number of times of blood pressure measurement performed by the blood pressure measurement unit in a predetermined time period in a prescribed time slot measured by the timer.

Accordingly, a count result of the number of times of blood pressure measurement in a prescribed time slot or in a predetermined time period in a prescribed time slot can be known.

Preferably, the prescribed time slot indicates a prescribed time slot before sleeping or a prescribed time slot after waking up, and thus a count result of the number of times of blood pressure measurement according to a lifestyle can be informed.

Preferably, the informing unit informs a difference between a required number of times of measurement designated beforehand and the number of times counted by the number of times of measurement count unit.

Accordingly, by designating the daily number of times of measurement and informing how many times are left to reach the designated number of times of measurement using the difference, the electronic blood pressure monitor can provide confirmation and guidance on blood pressure measurement, leading to improved compliance with blood pressure measurement. Further, since the electronic blood pressure monitor can inform the subject of how many times are left to reach the designated number of times of measurement using the difference and urge the subject to perform measurement, there is no need to incorporate an automatic measurement function or the like. Consequently, the electronic blood pressure monitor can be implemented with a simpler configuration and with a reduced cost.

Preferably, the electronic blood pressure monitor further includes a receiving unit for the required number of times of measurement described above.

Preferably, when the number of times counted by the number of times of measurement count unit in a predetermined time period in a prescribed time slot is less than a required number of times of measurement designated beforehand for the time period, the number of times of measurement count unit initializes the counted number of times of blood pressure measurement.

Accordingly, when the number of times in the predetermined time period in the prescribed time slot does not reach the required number of times of measurement, counting operation can be repeated until the number of times in the predetermined time period in the prescribed time slot reaches the required number of times of measurement.

Preferably, the electronic blood pressure monitor further includes a display unit, and the informing unit displays the count result on the display unit. More preferably, data of the blood pressure calculated by the blood pressure calculation unit and the count result are displayed on the display unit. Accordingly, the count result can be displayed using the display unit for the data of the blood pressure, without providing a special display unit for displaying the count result.

Preferably, the count result indicates the number of times of blood pressure measurement, and the informing unit displays the number of times of blood pressure measurement on the display unit using regions substantially in the shape of a rod, or displays the number of times of blood pressure measurement on the display unit by arranging pictograms having an identical shape.

Preferably, the count result indicates the number of times of blood pressure measurement, and the informing unit informs the number of times of blood pressure measurement using the number of lit LEDs.

Preferably, the electronic blood pressure monitor further includes a sound output unit, and the informing unit informs the count result using sound via the sound output unit.

Preferably, the number of times of measurement count unit includes a determination unit determining, for each blood pressure measurement, whether or not to count the blood pressure measurement among the number of times of measurement.

### EFFECTS OF THE INVENTION

According to the present invention, since the count result of the number of times of blood pressure measurement is informed, a subject can utilize the result to confirm the number of times of measurement or to obtain guidance on the required number of times of measurement.

### BRIEF DESCRIPTION OF THE DRAWINGS

Fig. 1 shows a hardware configuration of an electronic blood pressure monitor in accordance with each embodiment.
Fig. 2 shows schematic contents of a memory in accordance with each embodiment.
Fig. 3 shows a functional configuration of the electronic blood pressure monitor in accordance with each embodiment.
Fig. 4 is a flowchart illustrating a blood pressure measuring operation in accordance with each embodiment.
Fig. 5 shows an example of contents of an area in a memory in accordance with a first embodiment.
Fig. 6 is a flowchart for displaying the number of times of blood pressure measurement in accordance with the first embodiment.
Fig. 7 shows display examples of the first embodiment.
Fig. 8 shows an example of contents of areas in a memory in accordance with a second embodiment.
Fig. 9 is a flowchart for displaying the number of times of measurement in accordance with the second embodiment.
Fig. 10 shows a display example in accordance with the second embodiment.
Fig. 11 is a flowchart for displaying the number of times of measurement in accordance with a third embodiment.
Fig. 12 shows a display example in accordance with the third embodiment.
Fig. 13 shows an example of contents of areas in a memory in accordance with a fourth embodiment.
Fig. 14 is a flowchart for displaying the number of times of measurement in accordance with the fourth embodiment.
Fig. 15 is a flowchart for displaying the number of times of measurement in accordance with the fourth embodiment.
Fig. 16 shows a display example in accordance with the fourth embodiment.
Fig. 17 shows another display example in accordance with the fourth embodiment.
Fig. 18 shows still another display example in accordance with the fourth embodiment.
Fig. 19 shows a display example in accordance with a fifth embodiment.

### DESCRIPTION OF THE REFERENCE SIGNS

201 blood pressure calculation unit, 211 memory storage unit, 231 memory read unit, 241 display control unit, 261 the number of times of measurement update unit, 271 the number of times display data generation unit.

### BEST MODES FOR CARRYING OUT THE INVENTION

Hereinafter, embodiments of the present invention will be described in detail with reference to the drawings. In the description below, identical parts and components are denoted by the same numerals. Since they are identical in name and function, detailed description thereof will not be repeated.

An electronic blood pressure monitor in accordance with each embodiment has a function of informing data resulting from the number of times of blood pressure measurement performed on a day, during a week, or the like. The informed data can be utilized to confirm the number of times of measurement on the day, during the week, or the like, or to obtain guidance on the required number of times of measurement.

### [Configuration]

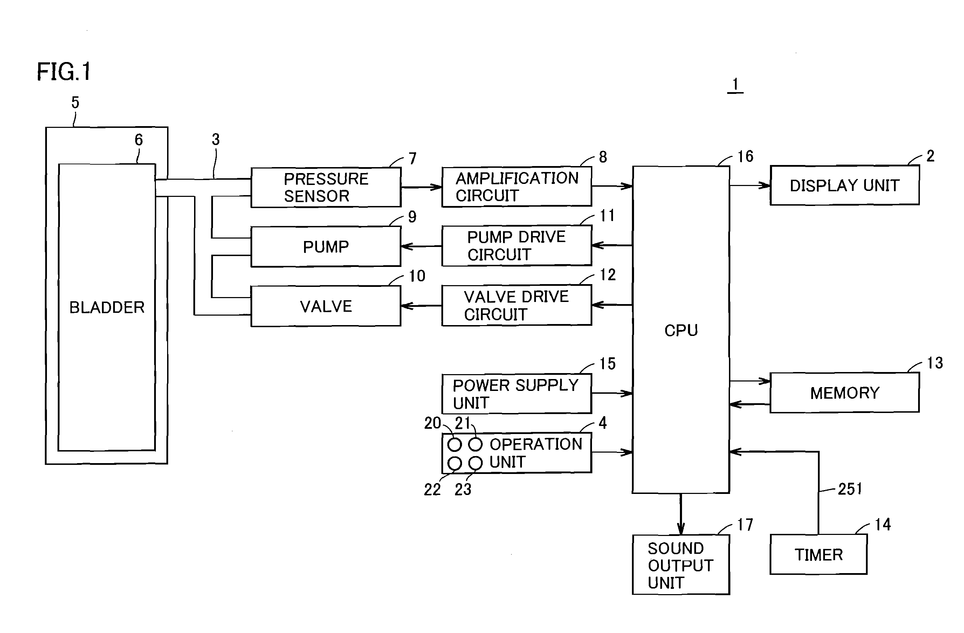

Fig. 1 shows a hardware configuration of an electronic blood pressure monitor in accordance with each embodiment.

Referring to Fig. 1, an electronic blood pressure monitor 1 includes a cuff 5 to be attached to a blood pressure measurement region of a subject (such as the subject's upper arm, wrist, or finger) for applying pressure to the measurement region by means of pneumatic pressure, and an air tube 3 connecting a main body of the blood pressure monitor with cuff 5. The main body of blood pressure monitor 1 includes a display unit 2 provided to allow the subject to confirm displayed contents, and an operation unit 4 provided to allow the subject to externally operate the blood pressure monitor.

Operation unit 4 has a power switch 20; a measurement switch 21 for designating start of pressurization, that is, start of measurement; a clock setting switch 22; and a memory call up switch 23 to be operated for calling up stored data (i.e., for reading data from a memory and displaying the read data on display unit 2). Power switch 20 is operated to turn on/off a power supply unit 15 which will be described later. Measurement switch 21 is operated to designate start or stop of blood pressure measurement.

Referring to Fig. 1, the main body of electronic blood pressure monitor 1 further includes: a pressure sensor 7 for outputting a change in a pulse pressure of the measurement region detected via a bladder 6 incorporated in cuff 5, as a pulse wave signal; an amplification circuit 8 amplifying a voltage signal indicating a pressure output from pressure sensor 7; a pump 9 and a valve 10 for adjusting a pressurization (air pressure) level of bladder 6; a pump drive circuit 11 driving pump 9; a valve drive circuit 12 for adjusting opening and closing of valve 10; a memory 13; a timer 14 operating to measure time and outputting measured time data 251; power supply unit 15; a sound output unit 17 for outputting sound; and a CPU (Central Processing Unit) 16 controlling and monitoring operation of each unit of the main body.

Measured time data 251 of timer 14 can be set by operating clock setting switch 22.

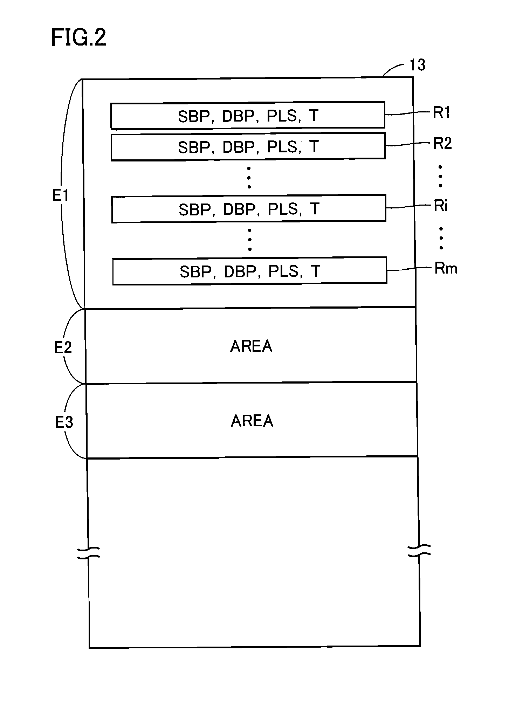

Referring to Fig. 2, memory 13 includes areas E1, E2, and E3. In area E1, measurement result data for each blood pressure measurement is stored in units of a record Ri (i = 1, 2, 3, ..., m), in a random manner or in order of a measurement time point. Record Ri includes systolic blood pressure data SBP indicating a systolic blood pressure, diastolic blood pressure data DBP indicating a diastolic blood pressure, pulse rate data PLS indicating a pulse rate, and time point data T indicating a measurement time point. These pieces of data may be stored in memory 13 in an associated manner every time when blood pressure measurement is performed, and the storage format is not limited to the one using record Ri. Time point data T is obtained based on measured time data 251 output by timer 14.

In the embodiments, area E 1 has a common configuration, whereas area E2 or E3 has a different configuration for each embodiment. Accordingly, details will be described later.

### [Functional Configuration]

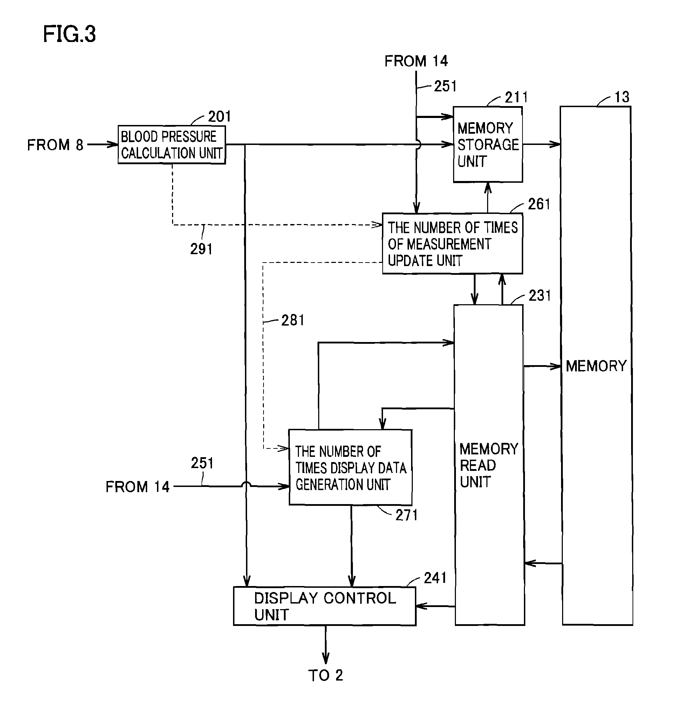

Fig. 3 shows a functional configuration of electronic blood pressure monitor 1 in accordance with each embodiment. Electronic blood pressure monitor 1 includes: a blood pressure calculation unit 201 for calculating a systolic blood pressure, a diastolic blood pressure, and a pulse rate based on the voltage signal supplied from amplification circuit 8; a memory storage unit 211 receiving data calculated by blood pressure calculation unit 201 (i.e., systolic blood pressure data SBP, diastolic blood pressure data DBP, and pulse rate data PLS) and measured time data 251 supplied from timer 14 to generate record Ri based on these pieces of received data, and storing the generated record Ri in memory 13; a memory read unit 231 reading data from memory 13; a display control unit 241 for displaying data on display unit 2; a number of times of measurement update unit 261 for updating the number of times of blood pressure measurement; and a number of times display data generation unit 271 generating data for displaying the number of times of measurement. Each of these units of Fig. 3 other than memory 13 may be prepared as a circuit, or prepared as a program stored in a prescribed area in memory 13. The function of each unit is implemented when the program corresponding to each unit is read from memory 13 and executed by CPU 16. Accordingly, CPU 16 functions as each unit of Fig. 3 other than memory 13.

### [Measurement Procedure]

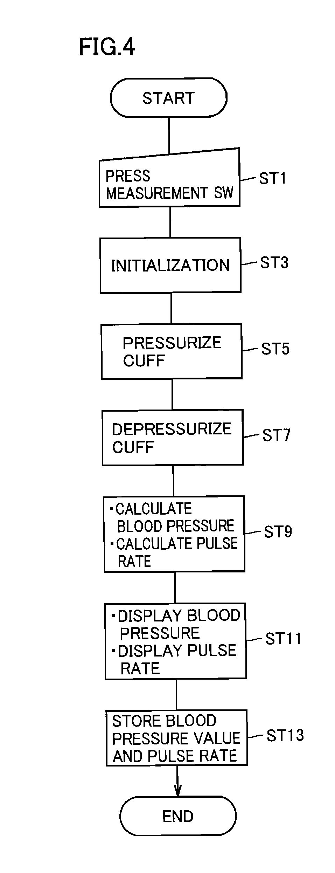

A blood pressure measuring operation in accordance with each embodiment will be described with reference to a flowchart of Fig. 4. A program according to the flowchart of Fig. 4 has been stored beforehand in a prescribed area in memory 13, and is read from memory 13 and executed by CPU 16. It is assumed that CPU 16 controls power supply unit 15 to supply power to each unit according to the operation of power switch 20 of electronic blood pressure monitor 1.

Firstly, the subject wraps cuff 5 around a measurement region and then operates measurement switch 21 (step ST (hereinafter simply referred to as ST) 1). As an initialization process for electronic blood pressure monitor 1, CPU 16 controls each unit to exhaust air from bladder 6 to effect correction to allow pressure sensor 7 to provide an output of 0 mmHg (ST3).

After the initialization process is finished, CPU 16 controls each unit to increase pressure inside bladder 6 up to approximately the systolic blood pressure of the subject + 40 mmHg (ST5), and thereafter gradually decreases the pressure inside bladder 6 (ST7). In this depressurization process, the pressure inside bladder 6 is detected by pressure sensor 7 and amplification circuit 8. Blood pressure calculation unit 201 calculates blood pressure (systolic blood pressure and diastolic blood pressure) values and a pulse rate based on the voltage signal output from amplification circuit 8, and temporarily stores the calculated blood pressure values and pulse rate in an internal memory of CPU 16 (ST9), and display control unit 241 displays the calculated blood pressure values and pulse rate on display unit 2 (ST11). Pressurization and depressurization process for blood pressure measurement is the same as that in a conventional electronic blood pressure monitor. Although blood pressure measurement is performed herein in the depressurization process, it may be performed in a pressurization process.

When the calculation and display of the blood pressure and the pulse rate is finished, memory storage unit 211 obtains time point data T based on measured time data 251 indicating a current time point received, generates record Ri including the obtained time point data T and data SBP, DBP, and PLS indicating the blood pressure (systolic blood pressure and diastolic blood pressure) values and the pulse rate read from the internal memory of CPU 16, and stores the generated record Ri in area E 1 of memory 13 (ST13). Thus, a sequence of blood pressure measurement is finished.

Although time point data T indicates the time when blood pressure measurement is finished as a time point for blood pressure measurement in the procedure of Fig. 4, time point data T may be data indicating the time when blood pressure measurement is started.

### [First Embodiment]

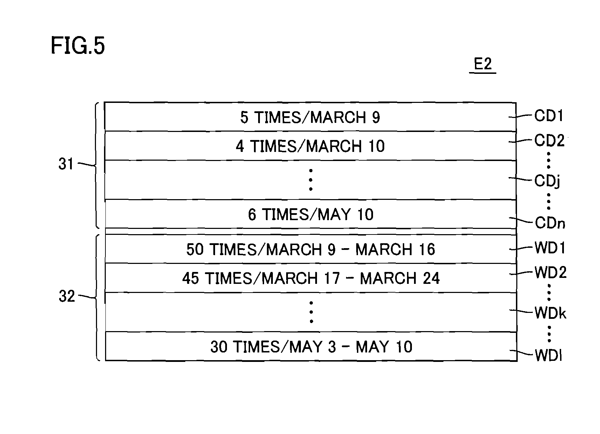

Fig. 5 shows an example of contents of area E2 in memory 13 in accordance with the present embodiment. In area E2, a times/day data group 31 and a times/week data group 32 are stored. Times/day data group 31 includes times/day data CDj (j = 1, 2, 3, ..., n). Times/day data CDj indicates each date and the number of times of blood pressure measurement performed on the date. Times/week data group 32 includes times/week data WDk (k = 1, 2, 3, ..., 1). Times/week data WDk indicates dates of each week and the number of times of blood pressure measurement performed during the week.

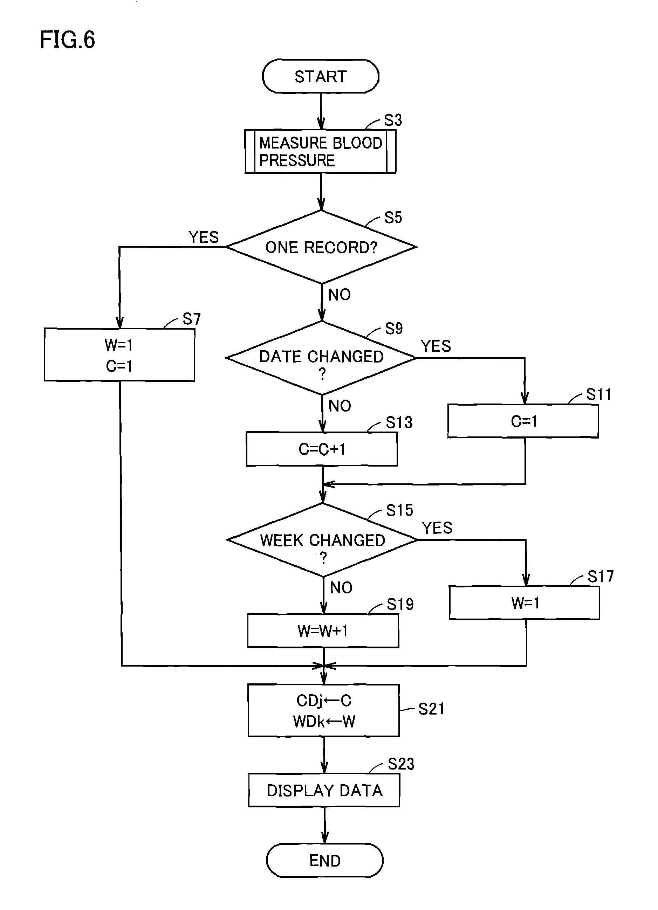

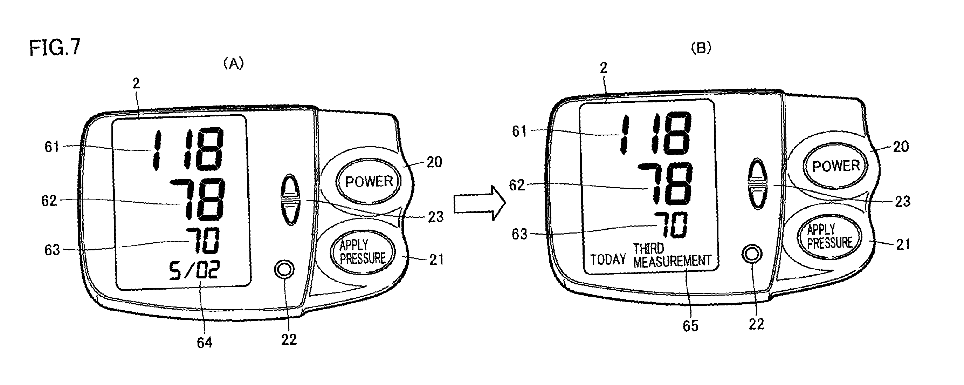

Fig. 6 shows a flowchart for displaying the number of times of blood pressure measurement in accordance with the present embodiment, and Fig. 7 shows display examples.

A program according to the flowchart of Fig. 6 has been stored beforehand in a prescribed area in memory 13. A function indicated by a procedure of Fig. 6 is implemented by CPU 16 reading the program from memory 13 and executing the program.

Referring to Fig. 6, the blood pressure measurement of Fig. 4 is performed (step S (hereinafter simply referred to as S) 3). Thereby, at least one record Ri is stored in area E1.

When the blood pressure measurement is finished, blood pressure calculation unit 201 provides the number of times of measurement update unit 261 with a notification 291 that the blood pressure measurement has been completed. Upon receiving notification 291, the number of times of measurement update unit 261 searches area E1 in memory 13 via memory read unit 231 to determine whether or not the number of record Ri stored therein is one (S5). When it is determined that the number of record Ri stored therein is one, the number of times of measurement update unit 261 sets 1 for each of temporary variables W and C (S7). Thereafter, the process proceeds to S21 which will be described later. Herein, temporary variable W is a variable for counting the number of times of blood pressure measurement performed during the current week, and temporary variable C is a variable for counting the number of times of blood pressure measurement performed on the current day.

On the other hand, when it is determined that the number of record Ri stored in area E1 is not one (NO in S5), that is, when it is determined that two or more records Ri are stored in area E1, the number of times of measurement update unit 261 determines whether or not the date has been changed based on time point data 251 supplied from timer 14 (S9). Specifically, the number of times of measurement update unit 261 stores time point data 251 in an internal memory (not shown) every time when it receives time point data 251, and when it receives time point data 251, it compares the received time point data 251 with the time point data stored beforehand in the internal memory, and determines whether or not the date has been changed based on a result of the comparison. When the number of times of measurement update unit 261 determines that the date has been changed (YES in S9), the number of times of measurement update unit 261 sets 1 for variable C (S11), and when the number of times of measurement update unit 261 determines that the date has not been changed (NO in S9), the number of times of measurement update unit 261 increments the value of variable C by 1 (S 13). Thereafter, the number of times of measurement update unit 261 determines whether or not the week has been changed based on a result of comparison between the received time point data 251 and time point data 251 stored beforehand in the internal memory (S15), as in the process in S9. When the number of times of measurement update unit 261 determines that the week has been changed (YES in S15), it sets 1 for variable W (S17), and when the number of times of measurement update unit 261 determines that the date has not been changed (NO in S15), it increments the value of variable W by 1 (S19). Thereafter, the process proceeds to the next process.

In the next process (S21), the number of times of measurement update unit 261 sets, for times/day data CDj, data indicating the date of today indicated by time point data 251 and the value of variable C, and stores times/day data CDj in area E2 via memory storage unit 211. The number of times of measurement update unit 261 also sets the value of variable W for times/week data WDk indicating the week indicated by time point data 251, and stores times/week data WDk in area E2 via memory storage unit 211. Thereby, when times/day data CDj indicating the date of today and times/week data WDk indicating dates of the current week have already been stored in area E2, these data are overwritten.

The number of times display data generation unit 271 displays data (S23). Specifically, when the number of times display data generation unit 271 receives from the number of times of measurement update unit 261 a notification 281 that update of the number of times of measurement in area E2 has been completed, it searches times/day data group 31 and times/week data group 32 for times/day data CDj indicating the date of today and times/week data WDk indicating the dates of the current week, respectively, to read each data, generates display data from the each read data, and supplies the generated display data to display control unit 241. Display control unit 241 displays the supplied data on display unit 2.

It is to be noted that the number of times display data generation unit 271 specifies the date of today and the dates of the current week based on time point data 251 supplied from timer 14, and searches area E2 via memory read unit 231 based on the specified data.

A display example (A) of Fig. 7 shows a state in which a systolic blood pressure data 61, a diastolic blood pressure data 62, a pulse rate data 63, and a measurement time point data 64 according to time point data 251 are displayed on display unit 2 in the process immediately after the blood pressure measurement (S3) is finished (ST11). A display example (B) of Fig. 7 is different from display example (A) in that measurement time point data 64 of display example (A) is replaced by the number of times data 65 indicating the number of times according to times/day data CDj of today, based on the procedure of Fig. 6. Although only the number of times data 65 according to times/day data CDj is displayed in display example (B), data according to times/week data WDk only may be displayed, or data according to both data may be displayed side by side.

While it has been described herein that the number of times of measurement on the day or during the week is automatically displayed subsequently to the blood pressure measurement (S3), display of the number of times of measurement on the day or the number of times of measurement during the week is not limited to the one performed subsequently to the blood pressure measurement (S3). For example, display state may be switched from the one shown in display example (A) to the one shown in display example (B) of Fig. 7 by the subject operating memory call up switch 23. That is, the processes from S5 onward of Fig. 6 may be started to be performed in response to the operation of memory call up switch 23.

### [Second Embodiment]

In the present embodiment, an electronic blood pressure monitor displaying the number of times of blood pressure measurement performed in the morning (for example, after waking up) and in the night (for example, before sleeping) will be described.

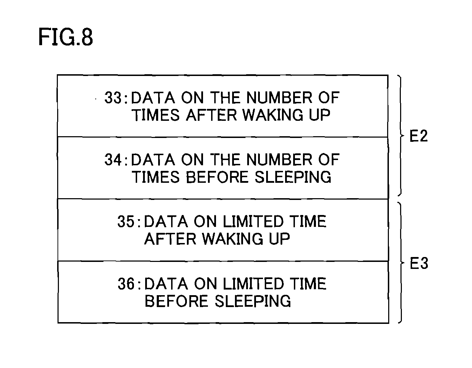

Fig. 8 shows an example of contents of areas E2 and E3 in memory 13 in accordance with the present embodiment. Data on the number of times after waking up 33 and data on the number of times before sleeping 34 are stored in area E2. Data on limited time after waking up 35 and data on limited time before sleeping 36 are stored in area E3. Data on the number of times after waking up 33 is data indicating the number of times of blood pressure measurement performed after waking up on the day. Data on the number of times before sleeping 34 indicates the number of times of blood pressure measurement performed before sleeping on the day. Data on limited time after waking up 35 is data indicating a limited time slot for blood pressure measurement after waking up, for example, from 6:00 to 8:00. Data on limited time before sleeping 36 is data indicating a limited time slot for blood pressure measurement before sleeping, for example, from 22:00 to 24:00. Data on limited time after waking up 35 and data on limited time before sleeping 36 may be stored beforehand in area E3 in a fixed manner, or may be configured to be variable for each subject (taking into account that the subject may be a shift worker).

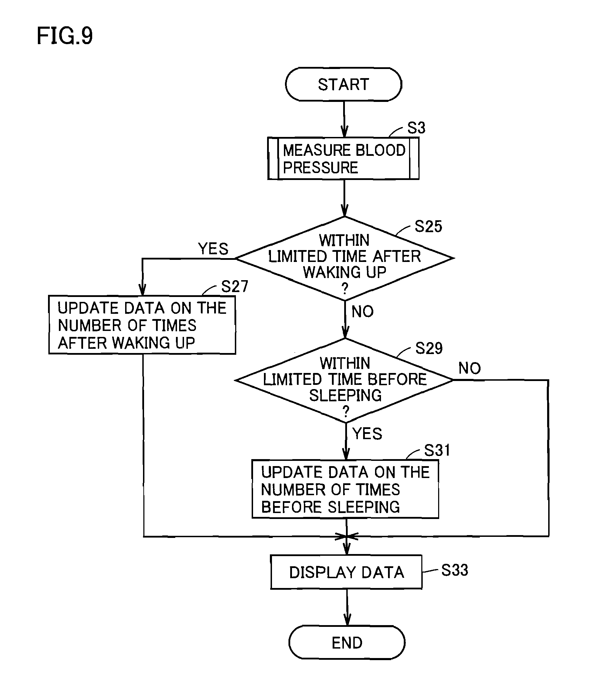

Fig. 9 shows a procedure for displaying the number of times of measurement in accordance with the present embodiment. A program according to a flowchart of Fig. 9 has been stored beforehand in a prescribed area in memory 13, and data display according to the procedure of Fig. 9 is implemented by CPU 16 reading the program from memory 13 and executing the read program. Although the number of times of measurement is automatically displayed also in the present embodiment every time when the blood pressure measurement (S3) described with reference to Fig. 4 is performed, display of the number of times of measurement may be started in response to external operation, as in the first embodiment.

In Fig. 9, firstly, the blood pressure measurement of Fig. 4 described above is performed (S3). After the measurement is finished, blood pressure calculation unit 201 provides the number of times of measurement update unit 261 with notification 291 that the blood pressure measurement has been completed. Upon receiving notification 291, the number of times of measurement update unit 261 determines whether or not this blood pressure measurement has been performed within limited time after waking up, based on time point data 251 supplied from timer 14 (S25). Specifically, the number of times of measurement update unit 261 compares the time point indicated by time point data 251 with a time slot indicated by data on limited time after waking up 3 5 read from area E3 via memory read unit 231, and determines whether or not the time point indicated by time point data 251 falls within the limited time slot indicated by data on limited time after waking up 35 based on a result of the comparison. When the number of times of measurement update unit 261 determines that the time point indicated by time point data 251 falls within the limited time slot (YES in S25), the number of times of measurement update unit 261 updates a value of data on the number of times after waking up 33 read from area E2 via memory read unit 231 by incrementing the value by 1, and stores the updated value in area E2 via memory storage unit 211 (i.e. overwrites previous data on the number of times after waking up 33) (S27). Thereafter, the process proceeds to S33 which will be described later.

On the other hand, when the number of times of measurement update unit 261 determines that the time point indicated by time point data 251 does not fall within the time slot indicated by data on limited time after waking up 35 (NO in S25), the number of times of measurement update unit 261 compares the time point indicated by time point data 251 with data on limited time before sleeping 36 read from area E2 via memory read unit 231, and determines whether or not the time point indicated by time point data 251 falls within a limited time slot indicated by data on limited time before sleeping 36 based on a result of the comparison (S29). When it is determined that the time point indicated by time point data 251 does not fall within the limited time slot (NO in S29), the process proceeds to S33 which will be described later. When it is determined that the time point indicated by time point data 251 falls within the limited time slot (YES in S29), the number of times of measurement update unit 261 increments data on the number of times before sleeping 34 read from area E3 via memory read unit 231 by 1, and stores the updated data on the number of times before sleeping 34 in area E2 via memory storage unit 211 (i.e. overwrites previous data on the number of times after waking up 34) (S31). Thereafter, the process proceeds to S33.

When the storage of the updated data is completed, the data on the number of times of measurement are displayed (S33). Specifically, when the processes in S25 to S31 are completed, the number of times of measurement update unit 261 provides the number of times display data generation unit 271 with notification 281 of completion. In response to receiving notification 281, the number of times display data generation unit 271 reads data on the number of times after waking up 33 and data on the number of times before sleeping 34 from area E2 via memory read unit 231, generates display data based on the read data, and supplies the generated display data to display control unit 241. Display control unit 241 displays the supplied data on display unit 2 (S33).

When the measurement time point does not fall within either of the time slot after waking up and the time slot before sleeping, data on the number of times after waking up 33 and data on the number of times before sleeping 34 are not updated.

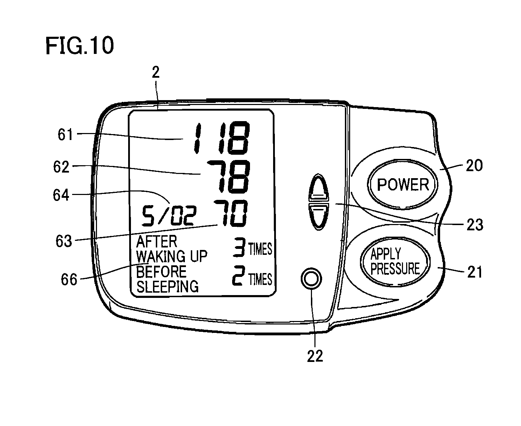

Fig. 10 shows a display example of S33. In Fig. 10, based on data of data on the number of times after waking up 33 and data on the number of times before sleeping 34, the number of times of blood pressure measurement performed after waking up on the day is indicated as "3 times", and the number of times of blood pressure measurement performed before sleeping on the day is indicated as "2 times".

Herein, only data on the number of times after waking up 33 and data on the number of times before sleeping 34 for one day are stored in area E2. Specifically, it is assumed that, on the next day (i.e., after 24 hours from the time point indicated by data on limited time after waking up 3 5 or the time point indicated by data on limited time before sleeping 35), data on the number of times after waking up 33 or data on the number of times before sleeping 34 is initialized by the number of times of measurement update unit 261 to be cleared (i.e., to have a value 0). This initialization may be performed as described below. That is, the time point when data on the number of times after waking up 33 or data on the number of times before sleeping 34 is counted up from 0 to 1 based on time point data 251 supplied from timer 14 has been stored distinctly based on time point data 251 supplied from timer 14, and is initialized when a difference between the stored time point and a time point indicated by time point data 251 which is sequentially received is approximately 24 hours.

Although it has been described in the present embodiment that data on the number of times after waking up 33 and data on the number of times before sleeping 34 are stored only in units of one day, it may be configured such that data on the number of times after waking up 33 and data on the number of times before sleeping 34 for each day are accumulated and stored in area E2.

### [Third Embodiment]

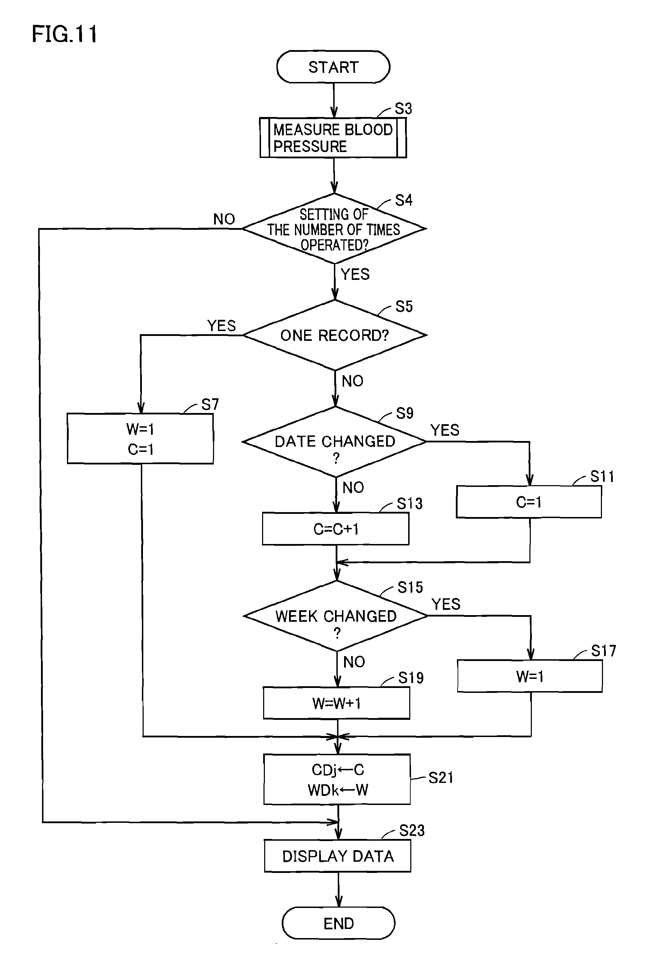

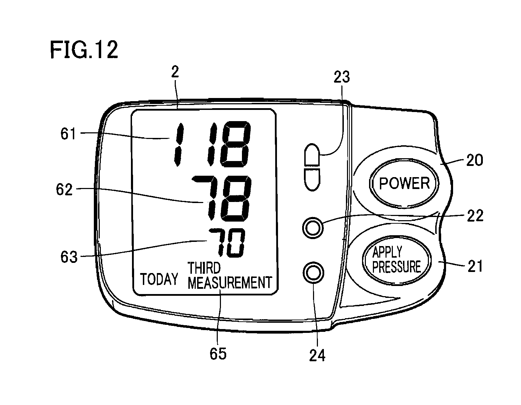

In the present embodiment, an electronic blood pressure monitor that allows a subject to freely set the number of times of blood pressure measurement will be described. Fig. 11 shows a flowchart for displaying data on the number of times of measurement in accordance with the present embodiment, and Fig. 12 shows a display example thereof. Referring to Fig. 12, a switch 24 for setting the number of times of measurement is additionally provided on operation unit 4.

The flowchart of Fig. 11 is identical to the flowchart of Fig. 6 shown in the first embodiment except that a determination process in S4 is added between the processes in S3 and S5 of the flowchart of Fig. 6. Accordingly, the process in S4 will be described, and description of other processes will not be repeated.

In the present embodiment, after the blood pressure measurement (S3) is finished, CPU 16 determines whether or not switch 24 for setting the number of times of measurement has been operated by the subject (S4). If it is determined that the switch has not been operated, although the blood pressure measurement has been performed this time, the number of times of measurement update unit 261 does not update the values of times/day data CDj of the day and times/week data WDk of the week. Thereafter, the process proceeds to S23. On the other hand, if it is determined that switch 24 has been operated (YES in S4), the processes from S5 onward are performed as described above.

Therefore, the subject can freely decide whether or not to reflect the result of the performed blood pressure measurement in the data on the number of times stored in memory 13.

Although it is described herein that switch 24 is separately provided and operated to set the number of times of measurement, the number of times of measurement may be set by operating other switches such as memory call up switch 23 in a combined manner, without providing separate switch 24.

### [Fourth Embodiment]

In the present embodiment, an electronic blood pressure monitor capable of informing a subject of the number of times of blood pressure measurement to be performed in the morning (for example, after waking up) and in the night (for example, before sleeping) will be described. In the present embodiment, a condition of a time slot such as after waking up (i.e., in the morning) and before sleeping (i.e., in the night) has been set beforehand in area E3 in the memory, and, based on the set condition of the time after waking up (time before sleeping), the number of times of measurement performed in the time slot is counted and displayed. These time slots may also be configured to be freely set by a subject, for a subject who is a shift worker, for example.

In the present embodiment, the number of times of measurement required to be performed after waking up (i.e., in the morning) and before sleeping (i.e., in the night) may be stored beforehand in area E3 in memory 13 in a fixed manner, or may be configured to be variable for each subject.

Further, in the present embodiment, when blood pressure measurement has been performed in the number of times not less than the number set in area E3 for each of the time slots after waking up (in the morning) and before sleeping (in the night), display of the number of times of measurement may maintain the last display state.

Furthermore, as to the measurement after waking up (in the morning) and before sleeping (in the night), whether or not the measurement has been performed in the required number of times within a prescribed time period (for example, within 10 minutes) in the time slot for measurement may be determined, and if not, the display of the number of times of measurement may be reset.

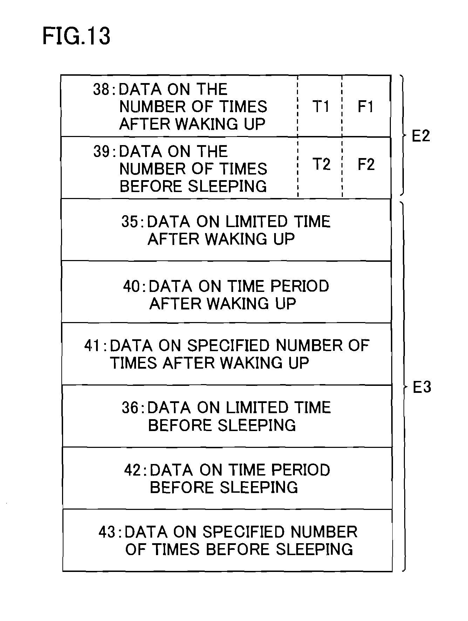

Fig. 13 shows an example of contents of areas E2 and E3 in memory 13 in accordance with the present embodiment. In area E2, data 38 indicating the counted number of times of blood pressure measurement after waking up and data 39 indicating the number of times of blood pressure measurement before sleeping are stored, and flags F1 and F2 and time point data T1 and T2 are also stored in a manner associated with data on the number of times after waking up 38 and data on the number of times before sleeping 39.

In area E3, data on limited time after waking up 35, data on a time period after waking up 40, and data on a specified number of times after waking up 41 are stored, and data on limited time before sleeping 36, data on a time period before sleeping 42, and data on a specified number of times before sleeping 43 are further stored. Data on the specified number of times after waking up 41 specifies the number of times of blood pressure measurement required within a prescribed time period (for example, 10 minutes) indicated by data on the time period after waking up 40 in a limited time slot indicated by data on limited time after waking up 35. Similarly, data on the specified number of times before sleeping 43 specifies the number of times of blood pressure measurement required within a prescribed time period (for example, 10 minutes) indicated by data on the time period before sleeping 42 in a limited time slot before sleeping indicated by data on limited time before sleeping 36.

Based on these data, it is designated that blood pressure measurement in the number of times indicated by data on the specified number of times after waking up 41 should be performed within any 10 minutes indicated by data on the time period after waking up 40, in a time slot indicated by data on limited time after waking up 35, for example from 6:00 to 8:00. Similarly, it is designated that blood pressure measurement in the number of times indicated by data on the specified number of times before sleeping 43 should be performed within any 10 minutes indicated by data on time period before sleeping 42, in a time slot indicated by data on limited time before sleeping 36, for example from 22:00 to 24:00.

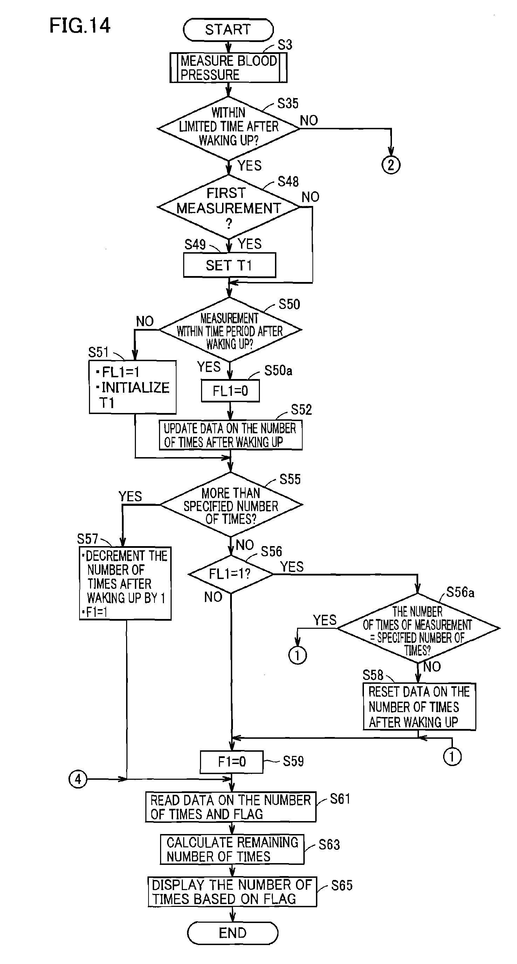

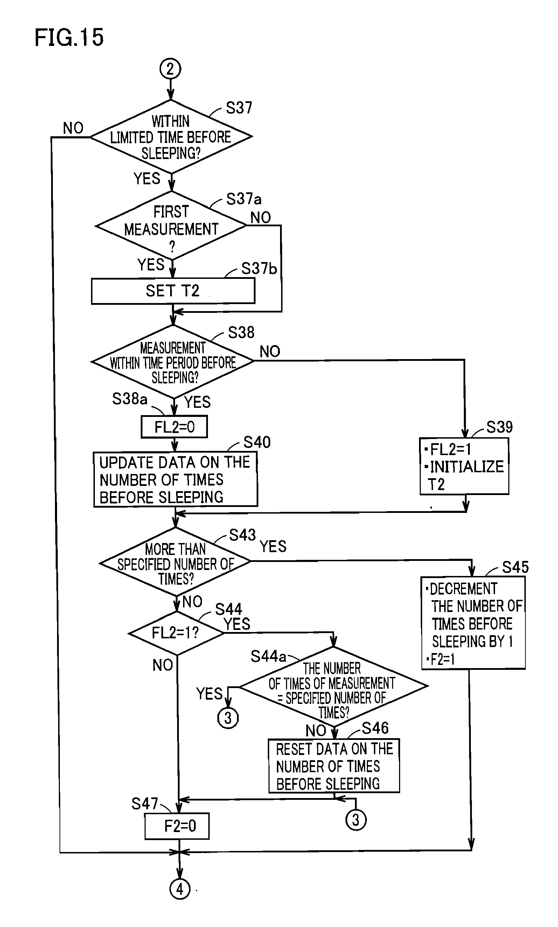

Figs. 14 and 15 show a process procedure in accordance with the present embodiment. A program according to a flowchart of Figs. 14 and 15 has been stored beforehand in a prescribed area in memory 13, and display processing in accordance with the present embodiment is implemented by CPU 16 reading the program from the area and executing the read program.

Referring to Fig. 14, firstly, the process of blood pressure measurement is performed (S3). Thereafter, the number of times of measurement update unit 261 receives from blood pressure calculation unit 201 notification 291 that the blood pressure measurement has been completed. Upon receiving notification 291, the number of times of measurement update unit 261 compares time point data 251 supplied from timer 14 with data on limited time after waking up 35 read via memory read unit 231, and determines whether or not a time point indicated by time point data 251 falls under data on limited time after waking up 35, based on a result of the comparison (S35). When the number of times of measurement update unit 261 determines that the current time point indicated by time point data 251 falls within a time slot designated for blood pressure measurement after waking up indicated by data on limited time after waking up 35 (YES in S35), the number of times of measurement update unit 261 subsequently determines whether or not updated data on the number of times after waking up 38 has a value "1" (S48). When the number of times of measurement update unit 261 determines that the updated data on the number of times after waking up 38 has a value "1" (YES in S48), the number of times of measurement update unit 261 stores time point data of the received time point data 251 in area E2 via memory storage unit 211, as data T1 (S49). Thereafter, the process proceeds to S50 which will be described later. When the number of times of measurement update unit 261 determines that the updated data on the number of times after waking up 38 does not have a value "1" (NO in S48), the process proceeds to S50. Data T1 indicates a time point at which the first blood pressure measurement within the prescribed time period indicated by data on the time period after waking up 40 was performed.

In the process in S50, the number of times of measurement update unit 261 reads data on the time period after waking up 40 via memory read unit 231, compares the received time point data 251 with the read data on the time period after waking up 40, and determines whether or not the time point indicated by time point data 251 falls within the prescribed time period indicated by data on the time period after waking up 40 (S50). When the number of times of measurement update unit 261 determines that the time point indicated by time point data 251 does not fall within the prescribed time period (NO in S50), the number of times of measurement update unit 261 sets 1 for a flag FL1 that is a temporary variable indicating that the time point does not fall within the prescribed time period, and initializes data T1 (S51). Thereafter, the process proceeds to S55 which will be described later. When the number of times of measurement update unit 261 determines that the time point indicated by time point data 251 falls within the prescribed time period (YES in S50), the number of times of measurement update unit 261 sets 0 for flag FL1 (S50a), reads data on the number of times after waking up 38 via memory read unit 231, increments the value of the read data on the number of times after waking up 38 by 1, and then stores the incremented data on the number of times after waking up 38 in area E2 via memory storage unit 211 (S52).

The determination process in S50 will be specifically described. If data on the number of times after waking up 38 in area E2 indicates "0", the number of times of measurement update unit 261 unconditionally determines that the time point indicated by time point data 251 falls within a time slot indicated by the read data on the time period after waking up 40 (YES in S50). In a case where data on the number of times after waking up 38 in area E2 indicates "1" or more, if a difference between the time point of data T 1 in area E2 and the time point indicated by the received time point data 251 is not more than the prescribed time period (for example, 10 minutes) indicated by data on the time period after waking up 40, the number of times of measurement update unit 261 determines that the time point indicated by time point data 251 falls within the time slot indicated by the read data on the time period after waking up 40 (YES in S50). If the difference is greater than the prescribed time period indicated by data on the time period after waking up 40, the number of times of measurement update unit 261 determines that the time point indicated by time point data 251 does not fall within the time slot indicated by the read data on the time period after waking up 40 (NO in S50).

Thereafter, the number of times of measurement update unit 261 determines whether or not the updated data on the number of times after waking up 38 has a value greater than the specified number of times indicated by data on the specified number of times after waking up 41 read from area E3 via memory read unit 231 (S55). When the number of times of measurement update unit 261 detects that blood pressure measurement has been performed in the number of times greater than the specified number of times based on a result of the determination (YES in S55), the number of times of measurement update unit 261 sets flag F1 read from area E2 via memory read unit 231 to "1", decrements the value of data on the number of times after waking up 38 by 1, and thereafter stores flag F1 set to 1 and the updated data on the number of times after waking up 38 in area E2 via memory storage unit 211 (i.e., overwrites previous data) (S57).

On the other hand, when the number of times of measurement update unit 261 determines that the updated data on the number of times after waking up 38 has a value within the specified number of times indicated by data on the specified number of times after waking up 41 (NO in S55), the number of times of measurement update unit 261 determines whether or not flag FL1 indicates "1" (S56). When the number of times of measurement update unit 261 determines that flag FL1=1 (YES in S56), it determines whether or not the number of times of measurement indicates the specified number of times (S56a). When it is determined that the number of times of measurement indicates the specified number of times (YES in S56a), the process proceeds to S59. When it is determined that the number of times of measurement does not indicate the specified number of times (NO in S56a), data on the number of times after waking up 38 is reset (i.e., a value 0 is set) (S58). Thereafter, the process proceeds to S59. When the number of times of measurement update unit 261 determines that flag FL1≠1 (NO in S56), the process proceeds to S59. In S59, the number of times of measurement update unit 261 sets flag F1 read from area E2 via memory read unit 231 to "0", and stores the set flag F1 and the updated data on the number of times after waking up 38 in area E2 via memory storage unit 211 (i.e., overwrites previous data) (S59).

After the process in S57 or S59, the number of times of measurement update unit 261 provides the number of times display data generation unit 271 with notification 281 that update has been completed. Therefore, processes from S61 onward are performed by the number of times display data generation unit 271.

Turning back to the process in S35, when the number of times of measurement update unit 261 receives notification 291 that the blood pressure measurement this time has been completed, and determines that the blood pressure measurement this time was not performed in the time slot indicated by data on limited time after waking up 35, (NO in S35), the process proceeds to S37 in Fig. 15.

In S37, the number of times of measurement update unit 261 determines whether or not the time point indicated by the received time point data 251 falls within a time slot indicated by data on limited time before sleeping 36 read from area E3 via memory read unit 231 (S37). When the number of times of measurement update unit 261 determines that the time point does not fall within the time slot (NO in S37), it provides the number of times display data generation unit 271 with notification 281 of completion, and thus the processes from S61 onward are performed by the number of times display data generation unit 271 and display control unit 241.

On the other hand, when the number of times of measurement update unit 261 determines that the blood pressure measurement this time was performed within the time slot indicated by data on limited time before sleeping 36 (YES in S37), the number of times of measurement update unit 261 subsequently determines whether or not updated data on the number of times before sleeping 39 has a value "1" (S37a). When the number of times of measurement update unit 261 determines that the updated data on the number of times before sleeping 39 has a value "1" (YES in S37a), the number of times of measurement update unit 261 stores the received time point data 251 in area E2 via memory storage unit 211, as data T2 (S3 7b), and thereafter, the process proceeds to S38. When the number of times of measurement update unit 261 determines that the updated data on the number of times before sleeping 39 does not have a value "1" (NO in S37a), the process proceeds to S38. Data T2 indicates a time point at which the first blood pressure measurement within the prescribed time period indicated by data on the time period before sleeping 42 was performed.

In the process in S38, the number of times of measurement update unit 261 determines whether or not the blood pressure measurement was performed within the prescribed time period indicated by data on the time period before sleeping 42 read from area E3 via memory read unit 231 (S38). When the number of times of measurement update unit 261 determines that the blood pressure measurement was not performed within the prescribed time period before sleeping (NO in S38), the number of times of measurement update unit 261 sets 1 for a flag FL2 that is a temporary variable indicating that the blood pressure measurement was not performed within the prescribed time period, and initializes data T2 (S39). Thereafter, the process proceeds to S43 which will be described later. When the number of times of measurement update unit 261 determines that the blood pressure measurement was performed within the prescribed time period (YES in S38), the number of times of measurement update unit 261 sets 0 for flag FL2 (S38a). Then, the number of times of measurement update unit 261 reads data on the number of times before sleeping 39 via memory read unit 231, increments the read data on the number of times before sleeping 39 by 1, and stores the incremented data on the number of times before sleeping 39 in area E2 via memory storage unit 211 (i.e., overwrites previous data) (S40).

The determination process in S38 will be specifically described. If the number of times of measurement update unit 261 determines that data on the number of times before sleeping 39 in area E2 indicates "0", the number of times of measurement update unit 261 unconditionally determines that the time point indicated by time point data 251 falls within a time slot indicated by the read data on the time period before sleeping 42 (YES in S38). In a case where the number of times of measurement update unit 261 determines that data on the number of times before sleeping 39 in area E2 indicates "1" or more, if a difference between the time point of data T2 in area E2 and the time point indicated by the received time point data 251 is not more than the prescribed time period (for example, 10 minutes) indicated by data on the time period before sleeping 42, the number of times of measurement update unit 261 determines that the time point indicated by time point data 251 falls within the prescribed time period indicated by the read data on the time period before sleeping 42 (YES in S38). If the number of times of measurement update unit 261 determines that the difference is greater than the prescribed time period indicated by data on the time period before sleeping 42, the number of times of measurement update unit 261 determines that the time point indicated by time point data 251 does not fall within the time slot indicated by data on the time period before sleeping 40 (NO in S38).

Thereafter, the number of times of measurement update unit 261 determines whether or not the number of times indicated by the read data on the number of times before sleeping 39 is greater than the number of times indicated by data on the specified number of times before sleeping 43 read from area E3 via memory read unit 231 (S43). When the number of times of measurement update unit 261 determines that the number of times indicated by data on the number of times before sleeping 39 is not more than the number of times indicated by data on the specified number of times before sleeping 43 (NO in S43), the number of times of measurement update unit 261 determines whether or not flag FL2 indicates "1" (S44). When the number of times of measurement update unit 261 determines that flag FL2=1 (YES in S44), it subsequently determines whether or not the number of times of measurement indicates the specified number of times (S44a). When it is determined that the number of times of measurement indicates the specified number of times (YES in S44a), the process proceeds to S47. When it is determined that the number of times of measurement does not indicate the specified number of times (NO in S44a), the value of data on the number of times before sleeping 39 is reset (i.e., 0 is set) (S46). Thereafter, the process proceeds to S47. When the number of times of measurement update unit 261 determines that flag FL2≠1 (NO in S44), the process proceeds to S47.

In S47, the number of times of measurement update unit 261 sets "0" for flag F2 read from area E2 via memory read unit 231, and stores the set flag F2 and the updated data on the number of times after waking up 38 in area E2 via memory storage unit 211 (i.e., overwrites previous data) (S47).

On the other hand, when the number of times of measurement update unit 261 determines that blood pressure measurement has been performed in the number of times greater than the specified number of times (YES in S43), the number of times of measurement update unit 261 sets flag F2 read from area E2 via memory read unit 231 to "1", decrements the value of data on the number of times before sleeping 3 9 by 1, and stores the set flag F2 and the updated data on the number of times before sleeping 39 in area E2 via memory storage unit 211 (i.e., overwrites) (S45).

After the process in S45 or S47, the number of times of measurement update unit 261 provides the number of times display data generation unit 271 with notification 281 that update has been completed. Therefore, the processes from S61 onward of Fig. 14 are performed by the number of times display data generation unit 271.

The processes from S61 onward will be described.

In response to receiving notification 281 that update of the number of times has been completed provided from the number of times of measurement update unit 261, the number of times display data generation unit 271 reads data on the number of times after waking up 38, data on the number of times before sleeping 39, and flags F1 and F2 from area E2, and data on the specified number of times after waking up 41 and data on the specified number of times before sleeping 43 from area E3, via memory read unit 231 (S61).

Thereafter, the remaining number of times of measurement to be performed is calculated for the number of times for each of the time slots before sleeping and after waking up (S63). Specifically, when flag F1 or F2 indicates "1", it means that blood pressure measurement in the specified number of times has been completed, and thus the remaining number of times of measurement to be performed before sleeping is determined as "0", or the remaining number of times of measurement to be performed after waking up is determined as "0". On the other hand, when flag F1 or F2 indicates "0", the remaining number of times of measurement to be performed is calculated according to (data on the specified number of times after waking up 41 - data on the number of times after waking up 38) or (data on the specified number of times before sleeping 43 - data on the number of times before sleeping 39).

Thereafter, display control unit 241 displays the number of times calculated in S63 for each of the time slots before sleeping and after waking up, based on the values of flags F1 and F2 (S65). Specifically, if flag F1 is "1", the number of times display data generation unit 271 instructs display control unit 241 to display an icon showing that blood pressure measurement in the specified number of times indicated by data on the specified number of times after waking up 41 has been completed. If flag F1 is "0", the number of times display data generation unit 271 generates display data indicating the calculated remaining number of times of measurement to be performed after waking up, and supplies the generated display data to display control unit 241. Similarly, if flag F2 is "1", the number of times display data generation unit 271 instructs display control unit 241 to display an icon showing that blood pressure measurement in the specified number of times indicated by data on the specified number of times before sleeping 43 has been completed. If flag F2 is "0", the number of times display data generation unit 271 generates display data indicating the calculated remaining number of times of measurement to be performed before sleeping, and supplies the generated display data to display control unit 241. Display control unit 241 displays data on the number of times on display unit 2, using data based on the instruction supplied from the number of times display data generation unit 271.

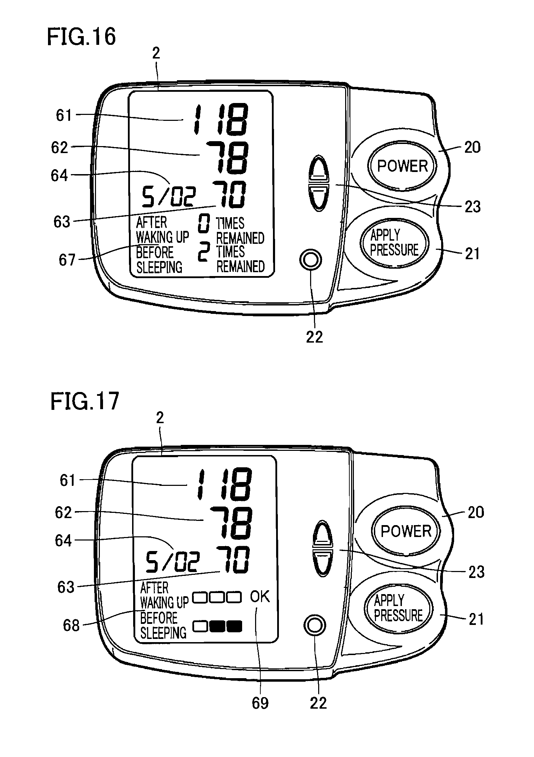

In Fig. 16, data 67 indicates that the remaining number of times of blood pressure measurement to be performed after waking up is 0 times, and the remaining number of times of blood pressure measurement to be performed before sleeping is two times.

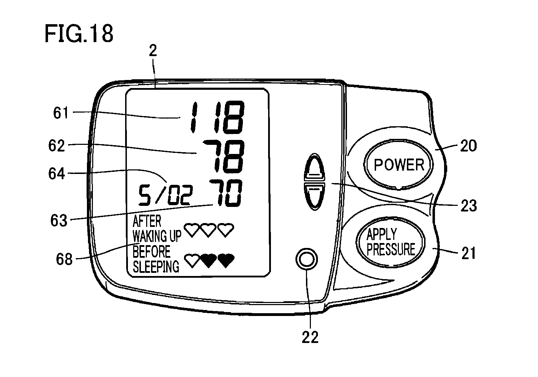

Further, as shown with data 68 in Fig. 17, the remaining number of times of measurement to be performed may be displayed using regions substantially in the shape of a rod, color change in pictograms, or solid black and hollow white marks. Data 68 indicates that blood pressure measurement after waking up has been completed in the specified number of times, and the remaining number of times of blood pressure measurement to be performed before sleeping is two times. When blood pressure measurement in the specified number of times has been completed, the completion of the measurement is indicated by, for example, a special ion 69. Further, instead of pictograms, light of an LED (light emitting diode) or the like may be used to inform the subject of the remaining number of times. Further, display as shown in Fig. 18 may be provided.

Although the remaining number of times of measurement to be performed is calculated and displayed for each of the time slots after waking up and before sleeping, the number of times of measurement to be performed per day may be set beforehand, and the remaining number of times of measurement to be performed on the day may be displayed. Further, the number of times of measurement to be performed per week may be set beforehand, and the remaining number of times of measurement to be performed during the week may be displayed.

### [Fifth Embodiment]

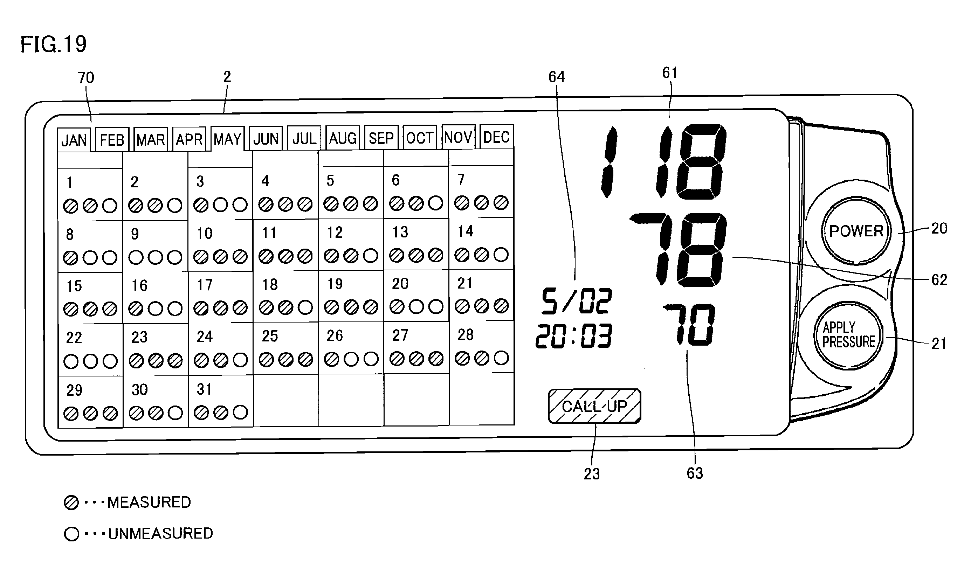

In the present embodiment, a calendar 70 may be displayed on display unit 2 as shown in Fig. 19, and the current number of times of measurement or the remaining number of times of measurement relative to the specified number of times of measurement may be displayed for each day on calendar 70.

In this case, data on the number of times after waking up 38 and associated flag F1, and data on the number of times before sleeping 39 and associated flag F2 are stored in area E2 to correspond to each day and month, according to the procedure in Figs. 14 and 15. Then, the processes in S61-S65 are performed on data for each day stored in area E2 according to data in area E3, and thus display as shown in Fig. 19 can be provided.

It is assumed that screen data for calendar 70 has been stored beforehand in a prescribed area in memory 13, and calendar 70 is displayed based on the screen data.

### [Sixth Embodiment]

Although it has been described in the above embodiments that data on the number of times of measurement is informed by displaying data on display unit 2, it may be informed using sound via sound output unit 17.

The embodiments disclosed herein are by way of example in all respects and should not be interpreted as restrictive. The scope of the present invention is determined not by the above description but by the appended claims, and intended to include all the modifications within the meaning and the scope equivalent to those of the claims.

## Claims

1. An electronic blood pressure monitor, comprising:
a blood pressure measurement unit including a cuff (5) to be attached at a region for measurement of blood pressure, a pressurization and depressurization unit (9, 10) adjusting pressure to be applied to said cuff, a pressure detection unit (7) detecting pressure inside said cuff adjusted by said pressurization and depressurization unit, and a blood pressure calculation unit (201) calculating blood pressure based on the pressure detected by said pressure detection unit;
a number of times of measurement count unit (261) counting a number of times of blood pressure measurement performed by said blood pressure measurement unit; and
an informing unit (271, 241) informing a count result counted by said number of times of measurement count unit.

2. The electronic blood pressure monitor according to claim 1, wherein said number of times of measurement count unit counts the number of times of said blood pressure measurement for each day.

3. The electronic blood pressure monitor according to claim 1, wherein said number of times of measurement count unit counts the number of times of said blood pressure measurement for each week.

4. The electronic blood pressure monitor according to claim 1, further comprising a timer for measuring time,
wherein said number of times of measurement count unit counts the number of times of the blood pressure measurement performed by said blood pressure measurement unit in a prescribed time slot measured by said timer.

5. The electronic blood pressure monitor according to claim 1, further comprising a timer for measuring time,
wherein said number of times of measurement count unit counts the number of times of the blood pressure measurement performed by said blood pressure measurement unit in a predetermined time period in a prescribed time slot measured by said timer.

6. The electronic blood pressure monitor according to claim 4 or 5, wherein said prescribed time slot indicates a prescribed time slot before sleeping.

7. The electronic blood pressure monitor according to claim 4 or 5, wherein said prescribed time slot indicates a prescribed time slot after waking up.

8. The electronic blood pressure monitor according to claim 1, wherein said informing unit informs a difference between a required number of times of measurement designated beforehand and the number of times counted by said number of times of measurement count unit.

9. The electronic blood pressure monitor according to claim 1, wherein, when the number of times counted by said number of times of measurement count unit in a predetermined time period in a prescribed time slot is less than a required number of times of measurement designated beforehand for the time period, said number of times of measurement count unit initializes said counted number of times of the blood pressure measurement.

10. The electronic blood pressure monitor according to claim 1, further comprising a display unit,
wherein said informing unit displays said count result on said display unit.

11. The electronic blood pressure monitor according to claim 1, further comprising a sound output unit,
wherein said informing unit informs said count result using sound via said sound output unit.

12. The electronic blood pressure monitor according to claim 1, wherein said number of times of measurement count unit includes a determination unit determining, for each blood pressure measurement, whether or not to count the blood pressure measurement among the number of times of measurement.

13. A method of informing a number of times of measurement in an electronic blood pressure monitor including a blood pressure measurement unit having a cuff (5) to be attached at a region for measurement of blood pressure, a pressurization and depressurization unit (9, 10) adjusting pressure to be applied to said cuff, a pressure detection unit (7) detecting pressure inside said cuff adjusted by said pressurization and depressurization unit, and a blood pressure calculation unit (201) calculating blood pressure based on the pressure detected by said pressure detection unit, comprising:
a number of times of measurement counting step counting a number of times of blood pressure measurement performed by said blood pressure measurement unit; and
an informing step informing a count result counted by said number of times of measurement counting step.
